# EUROPEAN PATENT APPLICATION

(11) **EP 4 442 260 A1**
(43) Date of publication of application: **09.10.2024**
(21) Application number: 23893664.5
(22) Date of filing: 13.11.2023
(51) Int. Cl.: A61K 31/41, A61P 35/00

(54) **USE OF BENZISOSELENAZOLE COMPOUND IN PREPARATION OF DRUG FOR TREATMENT OF SPINAGLIOMA**

(30) Priority: 21.11.2022 CN 202211460109
(71) Applicant: Beijing Neurosurgical Institute, Beijing 100070 (CN); Shanghai Yuanxi Medicine Corp, Shanghai 200000 (CN)
(72) Inventor: JIANG, Tao, Beijing 100070 (CN); ZENG, Fan, Beijing 100070 (CN); CHANG, Yuanhao, Beijing 100070 (CN); PANG, Bo, Beijing 100070 (CN); YIN, Hanwei, Shanghai 200000 (CN); ZENG, Huihui, Shanghai 200000 (CN)
(74) Representative: IPecunia
(86) International application number: PCT/CN2023/131184
(87) International publication number: WO 2024/109574

(57) **Abstract**

The present invention relates to use of a benzisoselenazole compound in the preparation of a drug for the treatment of spinaglioma. By applying the benzisoselenazole compound, cell proliferation of the spinaglioma can be effectively inhibited at the cellular level, with an obvious dose dependence. The tumor growth of a tumor-bearing mouse can be effectively inhibited at the animal model level, and the survival period of the tumor-bearing mouse can be prolonged.

## Description

### TECHNICAL FIELD

The present invention relates to the field of biomedicine, specifically to use of a benzisoselenazole compound in the preparation of a drug for the treatment of spinaglioma.

### BACKGROUND

Spinaglioma is a relatively common intraspinal tumor, and its incidence rate is only second to schwannoma and meningioma, accounting for 10-15% of the total spinal tumors. Primary spinagliomas account for approximately 80% of intramedullary tumors and can be classified according to their cellular origin. Among them, 60-70% are ependymomas, and 30-40% are astrocytomas, followed by angioblastoma and other rare lesions. The clinical manifestations of spinaglioma largely depend on the location and growth profile of the tumor. Most cases (about 70%) have pain as a main symptom, manifested as back pain, nerve root pain, or central nervous system pain, followed by sensory deficits (about 65%) and motor deficits (about 50%). Due to the non-specificity of symptoms, the average duration of symptoms before diagnosis is 3 years. Tumors occur almost evenly in cervical, thoracic, and lumbar segments. Among them, advanced tumors with higher malignancy and faster growth are prone to paralysis due to tumor compression.

At present, there is no clear optimal treatment means for high-grade spinaglioma with invasive growth. Medically commonly used imageology cannot distinguish different types of intramedullary tumors or other diseases such as myelitis, demyelination, and spinal cord infarction. Therefore, invasive methods such as biopsy are used clinically to clarify pathological diagnosis. However, unlike intracranial gliomas, the special functions of the spinal cord and its associated axonal fasciculi need maximum protection during surgery, making complete tumor resection almost impossible. In addition, the effects of radiotherapy and chemotherapy in the treatment of spinaglioma are still controversial. Although adjuvant radiotherapy or postoperative radiotherapy has been adopted, its exact therapeutic effect has not been systematically verified, and the use of radiotherapy and chemotherapy drugs has limited benefits for patients with spinaglioma. At present, temozolomide is used clinically to treat intracranial gliomas, but its indications do not include spinaglioma. Experimental chemotherapy with temozolomide has a poor effect on spinaglioma, and the temozolomide is highly resistant. Other anti-angiogenic drugs such as bevacizumab can only alleviate symptoms and cannot inhibit the progression of spinaglioma. Therefore, the development of new therapeutic drugs and means is imperative.

### SUMMARY

The present invention aims to develop a drug that has higher targeting specificity and can provide precise treatment for spinaglioma.

In order to achieve the above objective, the present invention provides use of a benzisoselenazole compound in the preparation of a drug for the treatment of spinaglioma, where a structure of the benzisoselenazole compound is as shown in formula (1),

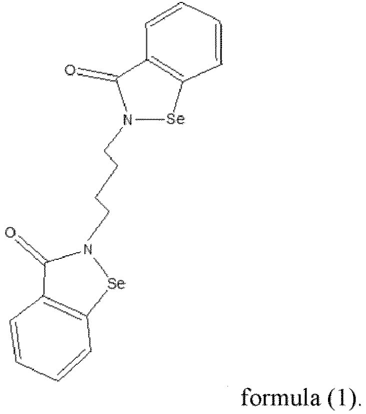

According to the present invention, the spinaglioma is WHO grade III spinaglioma or WHO grade IV spinaglioma; preferably, the spinaglioma is high-expression spinaglioma with TXNRD 1 protein, and in the high-expression spinaglioma with TXNRD1 protein, IHC staining scores of the TXNRD1 protein are 55-90.

According to the present invention, optionally, a dosage of the benzisoselenazole compound is 0.084-1119.58 mg/m², preferably 0.168-1017.80 mg/m².

According to the present invention, optionally, a content of the benzisoselenazole compound in the drug is 0.2-7.92 mg/mL, preferably 0.4-7.20 mg/mL.

According to the present invention, optionally, administration of the drug includes at least one of oral administration, intraperitoneal injection administration, and lumbar puncture intrathecal administration.

According to the present invention, optionally, the drug further includes auxiliary ingredients, which include at least one of hydroxypropyl-β-cyclodextrin, dimethyl-β-cyclodextrin, sulfobutyl-β-cyclodextrin, and Tween 80.

Optionally, a dosage form of the drug is an injection or an oral preparation.

On the other hand, optionally, the present invention further provides a drug for the treatment of spinaglioma, wherein the drug includes a benzisoselenazole compound as an active ingredient,
a structure of the benzisoselenazole compound is as shown in formula (1):

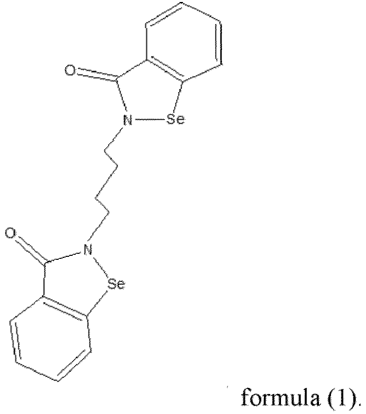

According to the present invention, optionally, a content of the benzisoselenazole in the drug is 0.2-7.92 mg/mL, preferably 0.4-7.20 mg/mL.

According to the present invention, optionally, the drug further includes auxiliary ingredients, which include at least one of hydroxypropyl-β-cyclodextrin, dimethyl-β-cyclodextrin, sulfobutyl-β-cyclodextrin, and Tween 80.

According to the present invention, optionally, a dosage form of the drug is an injection or an oral preparation.

Through the above technical solution, the present invention provides use of a benzisoselenazole compound in the preparation of a drug for the treatment of spinagliomas. By using the benzisoselenazole compound, cell proliferation of the spinagliomas can be effectively inhibited at both the cellular level and the animal model level, and the compound has higher targeting specificity, can achieve precise treatment of gliomas, has low drug toxicity, and is beneficial to long-term maintenance.

Other features and advantages of the present disclosure will be described in detail in the following specific embodiments.

### Biological material deposit information

The biological deposit information involved in the present invention includes: classification and name: human-derived cells; deposit institution: General Microbiology Center of the China Committee for Culture Collection of Microorganisms; postal code: 100101; address: No. 3, Courtyard 1, West Beichen Road, Chaoyang District, Beijing; deposit date: September 17, 2021; deposit number: CGMCC No. 23029.

Classification and name: human-derived cells; deposit institution:: General Microbiology Center of the China Committee for Culture Collection of Microorganisms; postal code: 100101; address: No. 3, Courtyard 1, West Beichen Road, Chaoyang District, Beijing; deposit date: September 17, 2021; deposit number: CGMCC No. 23028.

### BRIEF DESCRIPTION OF THE DRAWINGS

The accompanying drawings are intended to provide a further understanding of the present disclosure, constitute a part of the description, and are used for interpreting the present disclosure together with the following specific embodiments, rather than limiting the present disclosure. In the figures:
FIG. 1 shows the sensitivity of spinaglioma SP0001 to a temozolomide compound, measured in vitro after 24 hours.
FIG. 2 shows the sensitivity of spinaglioma SP0001 to a temozolomide compound, measured in vitro after 48 hours.
FIG. 3 shows the sensitivity of spinaglioma SP0001 to a benzisoselenazole compound, measured in vitro after 24 hours.
FIG. 4 shows the sensitivity of spinaglioma SP0001 to a benzisoselenazole compound measured in vitro after 48 hours.
FIG. 5 is an immunofluorescence staining map showing inhibitory effects of a benzisoselenazole compound with different concentrations on the growth of spinaglioma cells SP0001.
FIG. 6 is a fluorescence intensity analysis chart corresponding to immunofluorescence staining results of the benzisoselenazole compound with different concentrations on the growth of spinaglioma cells SP0001.
FIG. 7 shows tumor volume change curves of mice in experimental groups after administration.
FIG. 8 shows weight change curves of mice in each group in the administration process.
FIG. 9 is a histogram showing the weight change rate of mice in each experimental group after administration.
FIG. 10 shows the mean tumor volume and body weight of mice in each experimental group after 24 days of treatment.
FIG. 11 is an immunohistochemical staining map of TR protein in tissues of patients with spinaglioma.
FIG. 12 is an immunohistochemical staining score map of TR protein expression level.
FIG. 13 shows proliferation curves of SCA-S01 cells and SP0001 cells.
FIG. 14 shows the expression level of TR protein in cells detected by Western Blot.
FIG. 15 is an immunofluorescence staining map of Ki-67 in SP0001 and SCA-S01 cells.
FIG. 16 shows the inhibitory effect of a cytarabine compound on the proliferation of SCA-S01 cells, measured in vitro after 24 hours.
FIG. 17 shows the inhibitory effect of a cytarabine compound on the proliferation of SCA-S01 cells, measured in vitro after 48 hours.
FIG. 18 shows the inhibitory effect of a cytarabine compound on the proliferation of SCA-S01 cells, measured in vitro after 96 hours.
FIG. 19 shows the inhibitory effect of a cytarabine compound on the proliferation of SP0001 cells, measured in vitro after 24 hours.
FIG. 20 shows the inhibitory effect of a cytarabine compound on the proliferation of SP0001 cells, measured in vitro after 48 hours.
FIG. 21 shows the inhibitory effect of a cytarabine compound on the proliferation of SP0001 cells, measured in vitro after 96 hours.
FIG. 22 shows the inhibitory effect of a compound BS on the proliferation of SCA-S01 cells, measured in vitro after 24 hours.
FIG. 23 shows the inhibitory effect of a compound BS on the proliferation of SCA-S01 cells, measured in vitro after 48 hours.

### DETAILED DESCRIPTION

The specific embodiments of the present disclosure will be described in detail below with reference to the accompanying drawings. It should be understood that the specific embodiments described herein are merely used for illustrating and interpreting the present disclosure, rather than limiting the present disclosure.

On the one hand, the present invention provides use of a benzisoselenazole compound in the preparation of a drug for the treatment of spinaglioma, wherein a structure of the benzisoselenazole compound is as shown in formula (1),

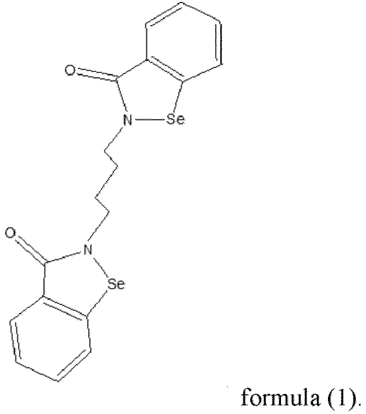

Optionally, the spinaglioma is WHO grade III spinaglioma or WHO grade IV spinaglioma; preferably, the spinaglioma is high-expression spinaglioma with TXNRD1 protein, and in the high-expression spinaglioma with TXNRD1 protein, IHC staining score of the TXNRD1 protein is 55-90.

Optionally, a dosage of the benzisoselenazole compound is 0.084-1119.58 mg/m², preferably 0.168-1017.80 mg/m².

Optionally, a content of the benzisoselenazole compound in the drug is 0.2-7.92 mg/mL, preferably 0.4-7.20 mg/mL.

Optionally, administration of the drug includes any one of oral administration, intraperitoneal injection administration, and lumbar puncture intrathecal administration.

Optionally, the drug further includes auxiliary ingredients, which include at least one of hydroxypropyl-β-cyclodextrin, dimethyl-β-cyclodextrin, sulfobutyl-β-cyclodextrin, and Tween 80.

Optionally, a dosage form of the drug is an injection or an oral preparation.

On the other hand, the present invention further provides a drug for the treatment of spinaglioma, wherein the drug includes a benzisoselenazole compound as an active ingredient,
a structure of the benzisoselenazole compound is as shown in formula (1):

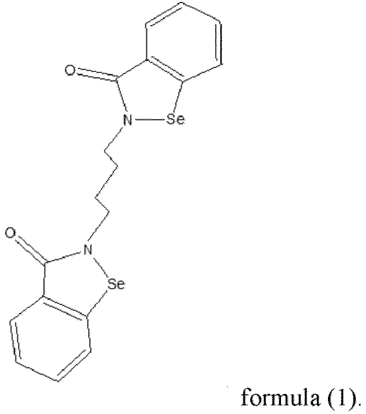

Optionally, a content of the benzisoselenazole in the drug is 0.2-7.92 mg/mL, preferably 0.4-7.20 mg/mL.

Optionally, the drug further includes auxiliary ingredients, which include at least one of hydroxypropyl-β-cyclodextrin, dimethyl-β-cyclodextrin, sulfobutyl-β-cyclodextrin, and Tween 80.

Optionally, a dosage form of the drug is an injection or an oral preparation.

The following further describes the present invention by examples, but the present invention is not limited thereby.

The spinaglioma cell SP0001 used in the present invention is a patient-derived spinaglioma cell with deposit number CGMCC No. 23029, and the spinaglioma cell SCA-S01 is a patient-derived spinaglioma cell with deposit number CGMCC No. 23028.

The benzisoselenazole compound used in the present invention was synthesized by entrusting Pharmaron (Beijing) New Drug Technology Co., Ltd. (857375-83-8); temozolamide was purchased from MedChemExpress; cytarabine CAS No.: 147-94-4.

### Example 1

This example used a temozolomide compound and a benzisoselenazole compound of the present invention for spinaglioma cell proliferation inhibition experiments.

Spinaglioma cells SP0001 in a logarithmic growth phase were digested with trypsin and centrifuged to obtain a cell precipitate, and cells were counted after being re-suspended in a complete medium. Based on the count results, the cells SP0001 were inoculated into a 96-well cell culture plate with 5000 cells per well and cultured in a 37°C incubator in a 5% CO₂ environment. After 24 hours, the cells were treated with drugs. A temozolomide (TMZ) compound and a benzisoselenazole compound (BS) of the present invention were respectively dissolved in DMSO to prepare a stock solution, which was then dissolved with the complete medium to different drug concentrations. The gradient concentrations of TMZ were 0, 100, 150, 200, 250, 300, 350, and 400 µM. The gradient concentrations of BS were 0, 1, 2, 3, 4, 5, 6, 7, and 8 µM. The medium was removed from the wells, and the corresponding drug solution of each concentration was added with 100 µL per well.

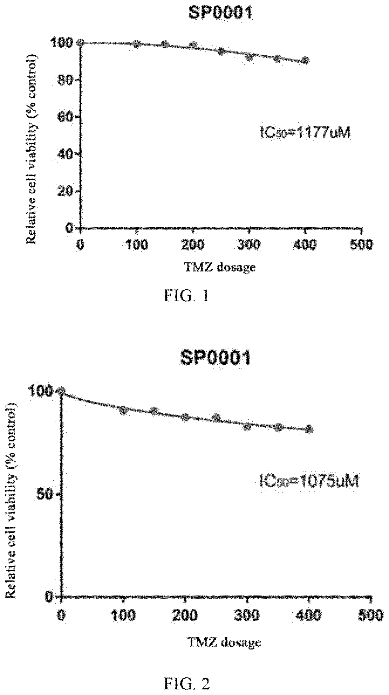

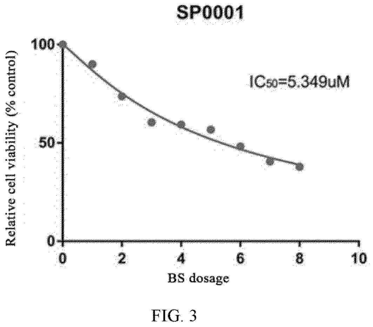

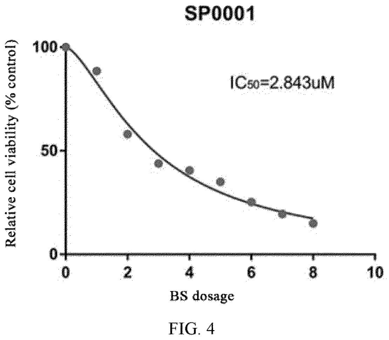

The well plate was put back into the incubator, and cell proliferation was tested after 24 and 48 hours, respectively. When testing, a CCK8 working solution was prepared in advance, namely, an appropriate volume of working solution was prepared based on 100 µL serum-free medium and 10 µL CCK8 per well, and the working solution was placed in dark. The well plate was taken out of the incubator, the cell medium was removed from the wells to be tested, 110 µL of CCK8 working solution was added to each well, the incubation was continued for 2 hours, then the absorbance at 450 nm was measured on an enzyme-linked immunosorbent assay (ELISA) reader, an inhibition rate corresponding to each concentration was calculated, and IC₅₀ values at corresponding time were calculated using GraphPad 7.0 software. The results were shown in FIGs. 1-4.

From the results in FIGs. 1-4, it can be seen that the patient-derived spinagliomas was not sensitive to the TMZ but sensitive to the benzisoselenazole compound, and the benzisoselenazole compound had an obvious inhibitory effect on the proliferation of a patient-derived spinagliomas cell line.

### Example 2

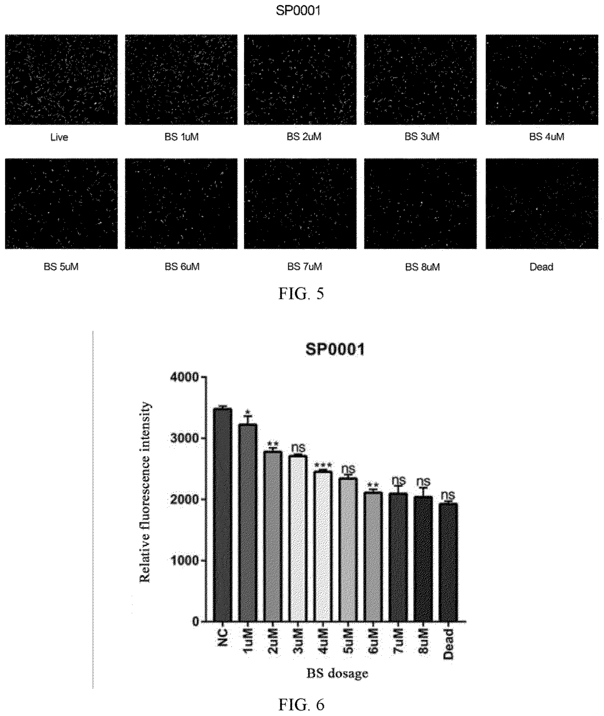

Fluorescence staining was performed using live and dead cell staining technology on the primary spinaglioma cell line SP0001 treated with the benzisoselenazole compound for 48 hours. The results were shown in FIGs. 5-6. As the drug concentration increased, the fluorescence intensity of live cells significantly weakened, and the killing effect of the 6 µM benzisoselenazole compound can reach 90%. The above experimental results proved that the benzisoselenazole compound can inhibit the proliferation of primary spinaglioma cells during in vitro experiment.

### Example 3

This example was used for explaining the inhibition of the benzisoselenazole compound on the growth of in vivo spinaglioma.

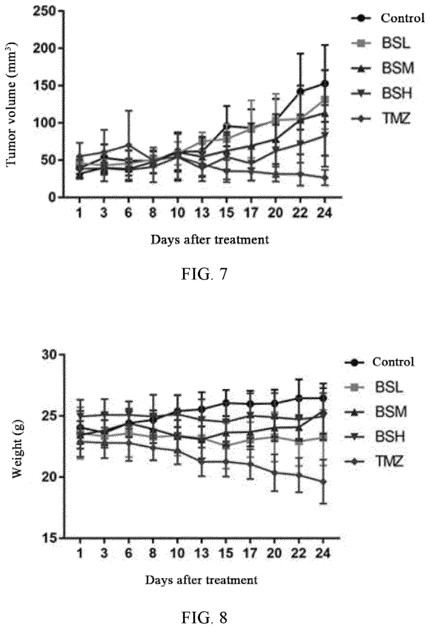

First, a tumor-bearing mouse model was built. Female NPG immunodeficient mice (6-8 weeks, starting weight 16-21 g, purchased from Beijing Vitalstar Biotechnology Co., Ltd.) were used for experimental research. The animals were raised and operated under sterile conditions, and all animal experiments were conducted in accordance with the international guidelines for animal experimentation. SP0001 human-derived spinaglioma tumor blocks (2-3 mm³) were prepared and inoculated subcutaneously to the right ventrodorsal side of each NPG mouse. After inoculation, the status of the mice was observed, and the size of the tumor was measured. The animals with a tumor volume of 40 mm³ were selected for experiment, and the animals that had too large tumor and no tumor were not selected. A total of 40 suitable tumor-bearing animals were selected for the experiment and divided into 5 experimental groups, with 8 mice in each group. The first day of administration was recorded as D1, the second day of administration was recorded as D2, and so on. All the animals were administered intragastrically once a day for 24 consecutive days. When the vital signs of the mice in the experimental groups were on the brink of death or their weight was lost significantly (> 20%), the experiment was terminated. When the experiment was terminated, the mice in all the experimental groups did not die. In the experiment, the physiological saline group was used as a negative control, and the temozolomide (TMZ) treatment group was used as a positive control. FIG. 7 shows change curves of tumor volume in NPG mice after administration.

During the experiment, no clinical abnormal symptoms or animal deaths due to drug toxicity were observed. FIG. 8 shows weight changes of mice in each group in the administration process. The BS groups (BSM and BSH groups) did not show any weight loss at this dose, indicating that the BS compound had no toxic and side effects on the NPG mice.

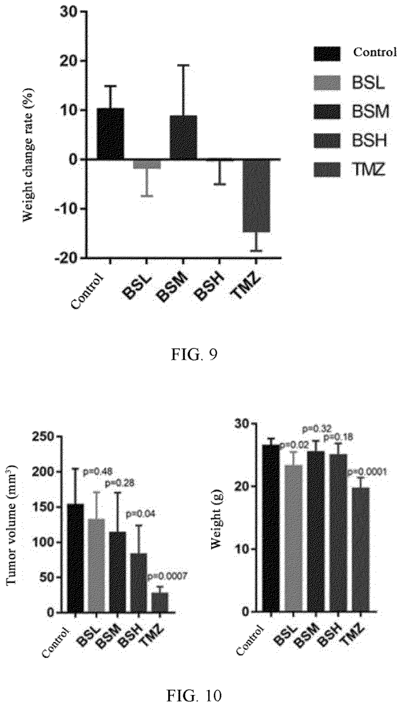

During the experiment, the TMZ group showed significant weight loss compared to the Control group, with a decrease of over 20%. The histogram of the weight change rate of NPG mice after administration was shown in FIG. 9 (BSL, BSM, and BSH represent a low dose group 90 mg/Kg, a medium dose group 180 mg/Kg, and a high dose group 360 mg/Kg of the drug BS respectively). It can be seen that TMZ had significant toxic and side effects on the NPG mice.

The BSH dose group and the TMZ group showed significant tumor inhibition, as shown in FIG. 10. At the end of the experiment, the tumor sizes of the Control group, BS (BSL, BSM, and BSH) groups, and TMZ group were 152.86 ± 51.53, 131.44 ± 39.81, 113.29 ± 57.21, 82.56 ± 41.40, and 26.61 ± 10.30 mm³, respectively, indicating that both the BS compound and the TMZ compound can inhibit the growth of spinaglioma. The body weights of the Control group, BS (BSL, BSM, and BSH) groups, and TMZ group were 26.46 ± 1.20, 23.22 ± 2.27, 25.42 ± 1.86, 24.96 ± 1.91, and 19.62 ± 1.79 mm³, respectively, further proving that the TMZ significantly reduced the body weight of mice and had significant toxic and side effects.

It can be seen that the BS (BSM and BSH) groups had no significant toxic and side effects on the NPG immunodeficient mice and exhibited a dependence on drug concentration dose. At the end of the experiment, the tumor volume inhibition rates of the BS low, medium, and high dose groups were 14.01%, 25.89%, and 45.99%, respectively, in dose dependence; the tumor volume inhibition rate of the TMZ group (30 mg/Kg) was 82.59%, indicating that the two drugs can effectively inhibit the growth of mouse tumors. The mice in the BS medium dose and high dose groups (BSM and BSH) showed no significant change in body weight and good mental status, indicating that the drug had low toxicity and was beneficial to long-term maintenance. However, the mice in the TMZ group showed significant weight loss, indicating that the TMZ had obvious toxicity.

### Example 4

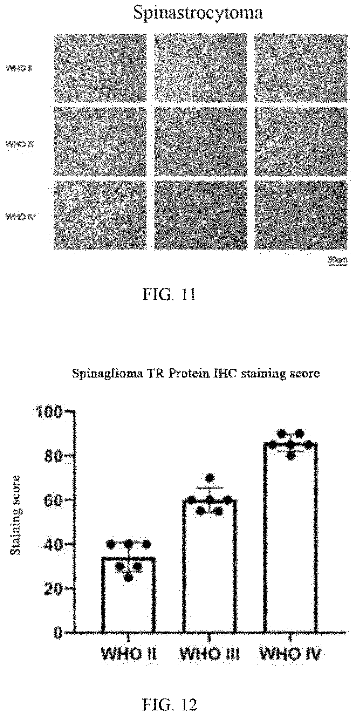

This example measured the malignancy degrees of two spinaglioma cell lines SCA-S01 and SP0001, respectively.
(1) TR protein (TXNRD1 protein) was a target of the compound BS of the present invention, and the compound BS of the present invention can achieve precise treatment of patients. In addition, the protein can be used as a biomarker for screening and prognosis of treated patients, with clinical application value. Immunohistochemical staining of the TR protein in the tissues of patients with spinaglioma was shown in FIG. 11; and immunohistochemical staining scores of the TR protein expression level were shown in FIG. 12. It was found in immunohistochemical experiments on tumor tissues of spinaglioma patients that the expression of the TR protein was significantly increased in high-grade spinaglioma patients. The IHC staining scores of TXNRD1 protein in WHO grade II spinaglioma were 25-40; the IHC staining scores of TXNRD1 protein in WHO grade III spinaglioma were 55-70; the IHC staining scores of TXNRD1 protein in WHO grade IV spinaglioma were 80-90; the IHC staining scores of TXNRD1 protein in high-expression spinagliomas with TXNRD1 protein (high-grade spinaglioma patients were WHO grade III or WHO grade IV spinagliomas patients) were 55-90.

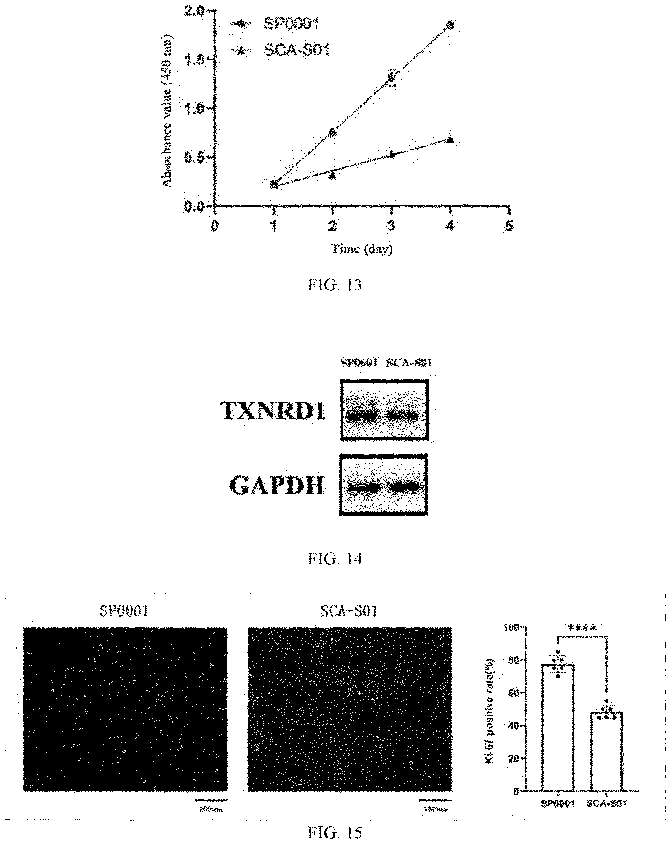

The present invention used two types of spinaglioma cells and compared their malignancy, wherein their deposit numbers were CGMCC No. 23028 (suspended cells, labeled as SCA-S01 in the figures) and CGMCC No. 23029 (adherent cells, labeled as SP0001 in the figures). First, proliferation curves of the cells were drawn by a CCK8 method to evaluate the proliferation abilities of the two types of cells. The results showed: within the same culture time, the proliferation of SP0001 cells was significantly higher than that of SCA-S01, indicating that the SP0001 cells had a higher cell proliferation rate and a higher degree of malignancy. The specific experimental method was as follows: SCA-S01 cells and SP0001 cells in a logarithmic growth phase were digested with an Accutase solution and a trypsin respectively to obtain cell suspensions, and the cells were inoculated into a 96-well cell culture plate with a quantity of 5000 cells per well. Cell proliferation was tested at 24, 48, 72, and 96 hours after inoculation. When testing, a CCK8 working solution was prepared in advance and placed in dark. The well plate was taken out of the incubator, the medium was removed for the wells to be tested, the CCK8 working solution was added, and the working solution was also added to blank wells of the well plate to test background absorbance. The well plate was put back into the incubator to inoculate the cells for 2 hours, then the well plate was taken out, 110 µL of the CCK8 working solution after inoculation was taken out from each well, the absorbance at 450 nm was tested on an ELISA reader, and cell proliferation curves were drawn, as shown in FIG. 13.

Total proteins of the SCA-S01 cells and the SP0001 cells were extracted, and the expression levels of the TR protein in the cells were tested by Western Blot assay. The results were shown in FIG. 14, where the expression level of the TR protein was higher in the SP0001 cells, indicating a higher degree of malignancy in the SP0001 cells.

(2) Experimental method: 24-well plates and matching 14 mm glass slides were used. 12 glass slides were disinfected with 75% ethanol, washed 3 times with PBS, and placed in the 24-well plates. 1 mL of polylysine was added to each well, and the well plates were placed overnight in a 37°C incubator. SP0001 and SCA-S01 cells were digested, re-suspended, counted, added to 12 wells with 10⁵ cells per well and 6 wells for each type of cells, and placed overnight in the 37°C incubator. The medium was discarded from the well plates, the well plates were washed 3 times with cold PBS for 5 minutes each time, 1 mL of cold 4% paraformaldehyde was added for fixation, and the cells were placed at room temperature for 10 minutes. The well plates were washed 3 times with cold PBS for 5 minutes each time, 1 mL of cold 0.3% Triton was added for membrane breaking treatment, and the cells were placed at room temperature for 15 minutes. The well plates were washed 3 times with cold PBS for 5 minutes each time, 5% BSA was added for closing, and the cells were placed at room temperature for 1 hour. 100 µL of Zhongshan Jinqiao mouse ki-67 primary antibody was added to each well, and the cells were placed overnight at 4°C. The primary antibody was discarded, the well plates were washed 3 times with cold PBST for 5 minutes each time, 100 µL of 594-APC fluorescent secondary antibody (abcam, 1:100) was added to each well, and the cells were placed at room temperature for 1 hour. The well plates were washed 3 times with cold PBST for 5 minutes each time. The glass slides were taken out, 2 drops of DAPI + mounting agent mixture was added to the glass slides, and the cells were covered with the glass slides to exhaust air, placed at room temperature in dark for 3 hours, and shot with an upright fluorescence microscope. The results were shown in FIG. 15 (dots in the fluorescence microscope map represent nuclei and ki-67; the histogram shows ki-67 positive rates of the two types of cells, and **** represents p < 0.0001). Ki-67 immunofluorescence staining on the two types of cells showed that SP0001 had higher Ki-67 expression than SCA-S01, further proving that SP0001 had a higher proliferation rate than SCA-S01 and a higher degree of malignancy.

### Example 5

This example measured the killing effects of a cytarabine compound and the BS compound of the present invention on two types of spinaglioma cell lines SCA-S01 and SP0001, using the same method as Example 1.

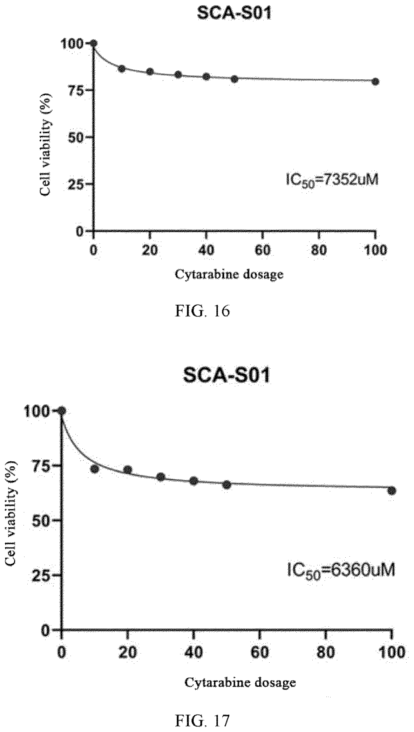

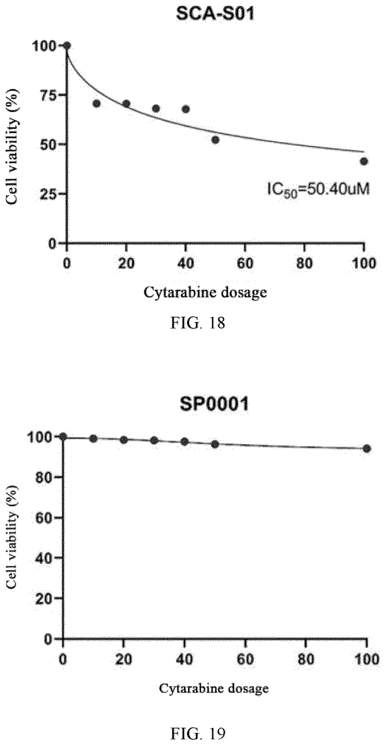

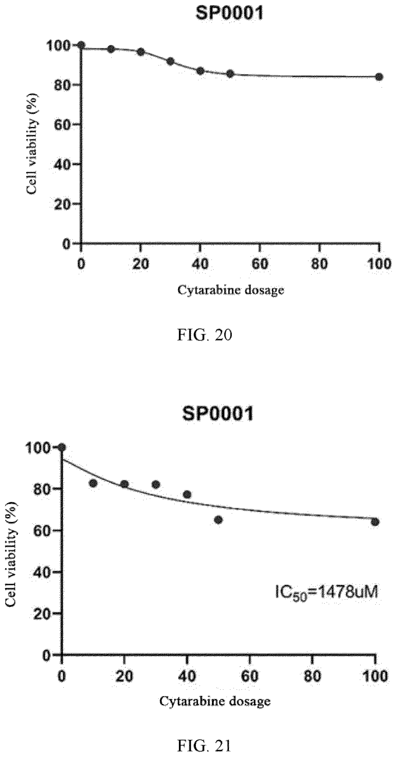

The inhibitory effects of the cytarabine compound on the proliferation of SCA-S01 cells (24 hours, 48 hours, and 96 hours) were shown in FIGs. 16-18, and its inhibitory effects on the proliferation of SP0001 cells (24 hours, 48 hours, and 96 hours) were shown in FIGs. 19-21.

The cytarabine had a weak killing effect on only SCA-S01 and cannot significantly inhibit the growth of SP0001 with higher malignancy at the same time point.

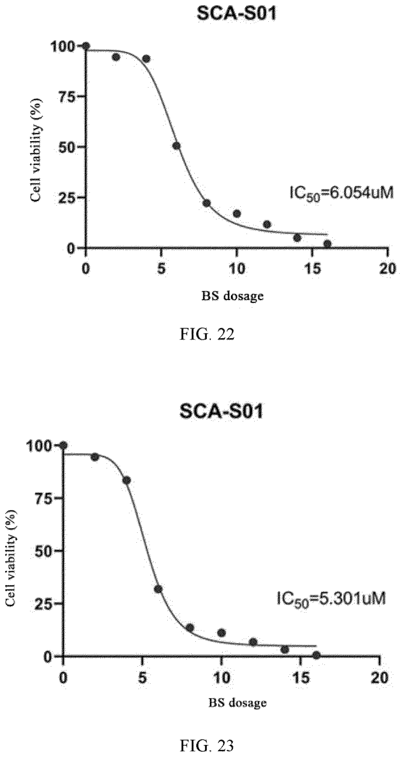

The killing effects of the BS drug on SCA-S01 cells (24 hours and 48 hours) were shown in FIGs. 22-23, and its killing effects on SP0001 cells were shown in Example 1.

In summary, the present invention provides use of a benzisoselenazole compound in the preparation of a drug for the treatment of spinagliomas. The results show that the benzisoselenazole compound has higher targeting specificity, strong killing effects on two types of existing human-derived spinaglioma cell lines, and a significant killing effect on SP0001 cells with higher malignancy. Therefore, the benzisoselenazole compound can be used to provide precise treatment for spinaglioma patients, has low drug toxicity, and is beneficial to long-term maintenance.

The preferred embodiments of the present disclosure are described in detail above with reference to the accompanying drawings. However, the present disclosure is not limited to the specific details in the above embodiments. Various simple variations may be made to the technical solutions of the present disclosure within the scope of the technical idea of the present disclosure, and these simple variations fall within the scope of the present disclosure.

It should be further noted that the specific technical features described in the above specific embodiments may be combined in any suitable manner without contradiction. In order to avoid unnecessary repetition, the present disclosure will not describe various possible combinations.

Moreover, the various different embodiments of the present disclosure may be combined randomly without deviating from the idea of the present disclosure, and the combinations should also be regarded as the disclosure of the present disclosure.

## Claims

1. Use of a benzisoselenazole compound in the preparation of a drug for the treatment of spinaglioma, wherein a structure of the benzisoselenazole compound is as shown in formula (1),

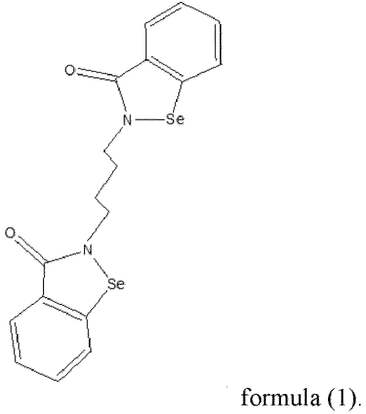

2. The use according to claim 1, wherein the spinaglioma is WHO grade III spinaglioma or WHO grade IV spinaglioma.

3. The use according to claim 1, wherein a dosage of the benzisoselenazole compound is 0.084-1119.58 mg/m²; and a content of the benzisoselenazole compound in the drug is 0.2-7.92 mg/mL.

4. The use according to claim 1, wherein administration of the drug comprises any one of oral administration, intraperitoneal injection administration, and lumbar puncture intrathecal administration.

5. The use according to claim 1, wherein the drug further comprises auxiliary ingredients, which comprise at least one of hydroxypropyl-β-cyclodextrin, dimethyl-β-cyclodextrin, sulfobutyl-β-cyclodextrin, and Tween 80.

6. The use according to claim 1, wherein a dosage form of the drug is an injection or an oral preparation.

7. A drug for the treatment of spinaglioma, wherein the drug comprises a benzisoselenazole compound as an active ingredient,
a structure of the benzisoselenazole compound is as shown in formula (1):

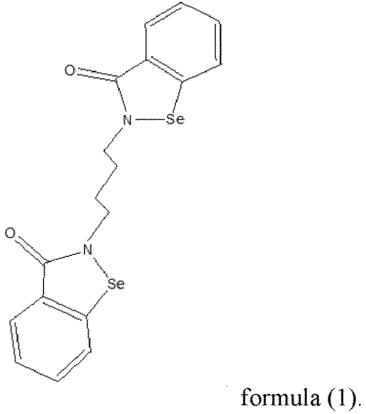

8. The drug according to claim 7, wherein a content of the benzisoselenazole in the drug is 0.2-7.92 mg/mL.

9. The drug according to claim 7, wherein the drug further comprises auxiliary ingredients, which comprise at least one of hydroxypropyl-β-cyclodextrin, dimethyl-β-cyclodextrin, sulfobutyl-β-cyclodextrin, and Tween 80.

10. The drug according to claim 7, wherein a dosage form of the drug is an injection or an oral preparation.
